(19) Europäisches Patentamt
European Patent Office
Office européen des brevets

(11) **EP 0 491 954 B1**

(12) **EUROPEAN PATENT SPECIFICATION**

(45) Date of publication and mention
of the grant of the patent:
**29.01.1997 Bulletin 1997/05**

(51) Int Cl.6: **D06H 3/08**, G01N 21/89,
G06T 7/40, D01G 31/00,
G01N 33/36

(21) Application number: **91909802.0**

(22) Date of filing: **28.05.1991**

(86) International application number:
**PCT/JP91/00709**

(87) International publication number:
**WO 91/19035 (12.12.1991 Gazette 1991/28)**

(54) **METHOD OF INSPECTING CLEANLINESS OF TOP AND DEVICE USED THEREFOR**

VERFAHREN UND VORRICHTUNG ZUM ERKENNEN DER SAUBERKEIT EINER OBERFLÄCHE

PROCEDE D'INSPECTION DE LA NETTETE DE LA SURFACE SUPERIEURE D'UN TISSU ET APPAREIL UTILISE A CET EFFET

(84) Designated Contracting States:
**CH DE FR IT LI**

(30) Priority: **28.05.1990 JP 139097/90**

(43) Date of publication of application:
**01.07.1992 Bulletin 1992/27**

(73) Proprietor: **KANEBO LTD.**
**Sumida-ku, Tokyo 131 (JP)**

(72) Inventors:
• **NISHI, Noriyuki**
**Jyoto-ku, Osaka-shi, Osaka 536 (JP)**
• **MUTO, Tadashi**
**Yamatokoriyama-shi, Nara 639-11 (JP)**
• **TAKAYAMA, Shinichi**
**Suzuka-shi, Mie 510-02 (JP)**

(74) Representative: **Pellmann, Hans-Bernd, Dipl.-Ing.**
**Patentanwaltsbüro**
**Tiedtke-Bühling-Kinne & Partner**
**Bavariaring 4**
**80336 München (DE)**

(56) References cited:
JP-A- 1 143 943          JP-A- 6 293 637
JP-A-63 273 014

**Description**

BACKGROUND OF THE INVENTION

Field of the Invention

The present invention relates to a method of and an apparatus for inspecting cleanness of natural fiber tops including wool and cotton for example.

Background Art

Normally, inspection of the cleanness of wool tops is executed in accordance with the inspection method prescribed in paragraph 5.6 (ruling the number of nep) and paragraph 5.7 (ruling the number of vegetal impurities) of JIS L-1083. The cleanness is designated by means of the number of detected defects per inspection item present in a predetermined amount of inspected top slibers.

Conventionally, cleanness of wool top sliber is visually inspected. Authorized inspectors visually detect and classify defects from wool top slibers aligned on an inspection table. Conventionally, a specific continuous inspection method has been executed in accordance with paragraphs 5.6 and 5.7 of JIS L-1083 by thinly and uniformly spreading top slibers in a gill box by about 0.5mm of thickness, and then, sampled top slibers are placed on an inspection table. Inspectors then visually count the number of impurities and defects by irradiating sampled top slibers with light beam permeated through an inspection window. Since inspectors are obliged to visually count the number of impurities and defects based on the conventional practice, results of the visual inspection are noticeably variable between inspectors.

In order to minimize the difference of the inspected results, only those strictly selected inspectors execute inspection of top slibers. Nevertheless, the resultant values are not yet reliable to full extent. Actually, the visually inspected results are merely considered to be relative values of comparison.

In order to fully solve this problem, the Applicant of the invention had previously filed an application for a patent as per the Japanese Patent Application No. 62-303563 which discloses a method of inspecting cleanness of top sliver comprising the steps of: generating an image signal by picking up an image of said top sliver with an image sensor; and discernibly classifying a defect into pillwise defect or vegetal defect mixed in said top sliver based on said generated image signal. In particular, the pillwise defects and the vegetal defects are classified by an elongation degree discrimination. This in turn obliges the proposed method to follow up complex computation to extract features of defects. As a result of slow speed available for the feature extracting process, the proposed method could not properly be applied to the execution of continuous inspection processes.

It is an object of the present invention to provide a method and an apparatus for inspecting the cleanness of top sliver with which the cleanness of top sliver can be inspected precisely and very quickly.

According to the present invention this object is achieved by a method of inspecting cleanness of top sliver comprising the steps of: generating an image signal by picking up an image of said top sliver with an image sensor; and discernibly classifying a defect into pillwise defect or vegetal defect mixed in said top sliver based on said generated image signal; said method being characterized in that in the course of classifying said defects, a membership function (solely available for conditional section) for dealing with features of classified defects and the other membership function (solely available for conclusion section) for determining the probability of being defect against said classified defects are established, wherein said method also establishes a fuzzy rule by applying said conclusion section determining probability of being defect against those classified defects; and wherein said method further classifies defects based on fuzzy reasoning derived from said membership function (solely available for said conditional section), the other membership function (solely available for said conclusion section), and said fuzzy rule.

According to the present invention this object is also achieved by an apparatus for inspecting cleanness of top sliver comprising: a gill box which uniformly spreads a top sliver and then externally delivers it; a plurality of guide rollers which respectively guide said spread top sliver; an illuminating unit which irradiates said spread top sliver with a light beam; an image sensor which picks up an image of said spread top sliver and generates an image signal; a contact-free electrostatic preventing unit which is installed in the vicinity of said plurality of guide rollers and eliminates static charge from said spread top sliver; and an image processing unit which processes said image signal generated by said image sensor; said apparatus being characterized in that in the course of classifying said defects, said image processing unit establishes a membership function (solely available for conditional section) for dealing with features of classified defects and the other membership function (solely available for conclusion section) for determining the probability of being defect against said classified defects, wherein said image processing unit also establishes a fuzzy rule by applying said conclusion section determining probability of being defect against those classified defects; and wherein said image processing unit further classifies defects based on fuzzy reasoning derived from said membership function (solely available for said conditional section), the other membership function (solely available for said conclusion section), and said fuzzy rule.

The present invention will be described hereinbelow by way of example and with reference to the accompanying drawings.

BRIEF DESCRIPTION OF THE DRAWINGS

Fig. 1 presents an overall perspective view of the top inspection apparatus embodied by the invention;

Fig. 2 is an explanatory view of the operation of the image processing unit of the top inspection apparatus embodied by the invention;

Fig. 3 to Fig. 7 respectively designate the method of processing image of inspected top slibers;

Fig. 8 presents an operating flowchart designating steps for processing image of inspected top slibers;

Fig. 9 presents an operating flowchart designating the flow of operations for classifying cleanness of the inspected top slibers shown in Fig. 8;

Fig. 10 to Fig. 13 are respectively explanatory views of a variety of defects and the method of measuring the amount of features of respective defects;

Fig. 14 through Fig. 20 respectively and graphically designate membership functions to determine the amount of the features of respective defects;

Fig. 21 through Fig. 28 respective and graphically designate membership functions to deal with respective defects;

Fig. 29 graphically explans the method of determining adaptability to the amount of features of respective defects;

Fig. 30a through 30c respectively explain the method of implementing superimposition; and

Fig. 31 presents an operating flowcart of sequential processes for classifying defects of cotton slibers.

Fig. 32 shows an image chart designating distribution of depthwise levels of picture elements on the line indicated by character A;

Fig. 33 designates the distribution of depthwise levels of pillwise defect;

Fig. 34 designates the distribution of depthwise levels of vegital defect;

DESCRIPTION OF THE PREFERRED EMBODIMENTS

Optimal form for embodying the Invention

Referring now more particularly to the accompanying drawings, detail of the method of and apparatus for inspecting cleanness of top sliber according to an embodiment of the invention is described below.

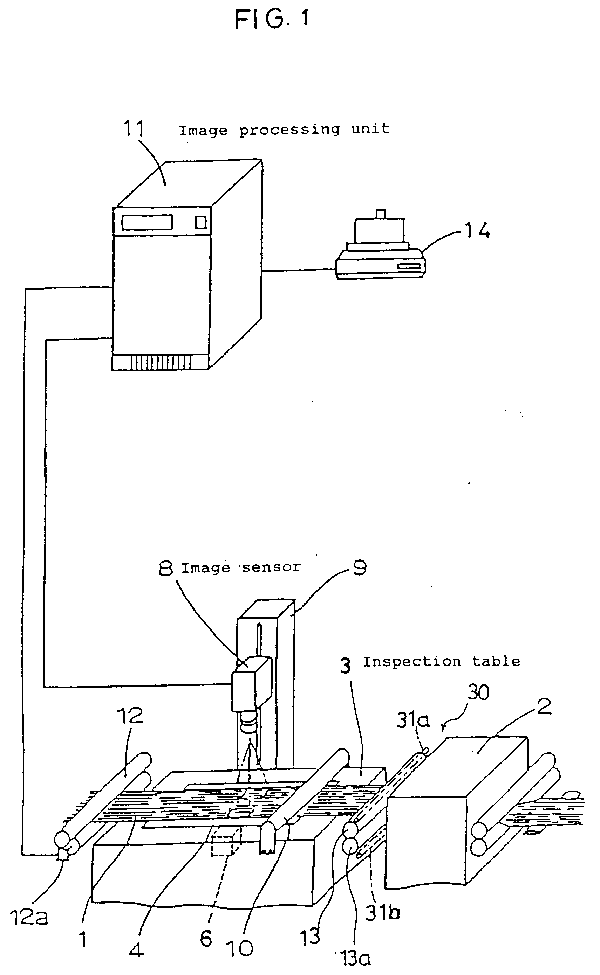

Fig. 1 presents an overall perspective view of the top cleanness inspection apparatus according to an embodiment of the invention. An image sensor (which is substantially a CCD camera) 8 held by a supporting rod 9 is secured to a position above an inspection window 4 of an inspection table 3. The image sensor 8 picks up image of sampled top sliber 1 which is continuously delivered from a gill box 2. An illuminating unit 6 installed below the inspection window 4 emits light beam upward. A roller 10 is installed in front of the image pickup position. The roller 10 prevents the top sliber 1 from superficially incurring fluffing symptom.

An electrostatic preveing unit 30 is provided in the vicinity of a pair of guide rollers 13 and 13a which are respectively installed to the outlet of the gill box 2. The electrostatic preventing unit 30 consists of a rod 31a which is opposite from the upper guide roller 13 and the other rod 31b being opposite from the lower guide roller 13b. A high voltage is delivered to these rods 31a and 31b to permit the needle tips of these rods to respectively execute corona discharge in order to ionize ambient air before eliminating static charge from the charged object in contact-free condition.

Next, image of the top sliber 1 picked up by the image sensor 8 is then converted into an image signal before being delivered to an image processing unit 11. Simultaneously, synchronizing signals needed for continuously picking up image are transmitted to the image processing unit 11 from a synchronizing sensor 12a secured to a measuring roller 12 which is set to a position close to the rear end of the inspection table 3.

The imagne processing unit 11 extracts a predetermined amount of features, and then, based on the extracted feature data, the image processing unit 11 classifies the inspected top sliber 1 into respective degrees of cleanness. Finally, the classified result is printed out by a printer 14 so that the result can be made available for the table designating concrete results of the inspection of cleanness.

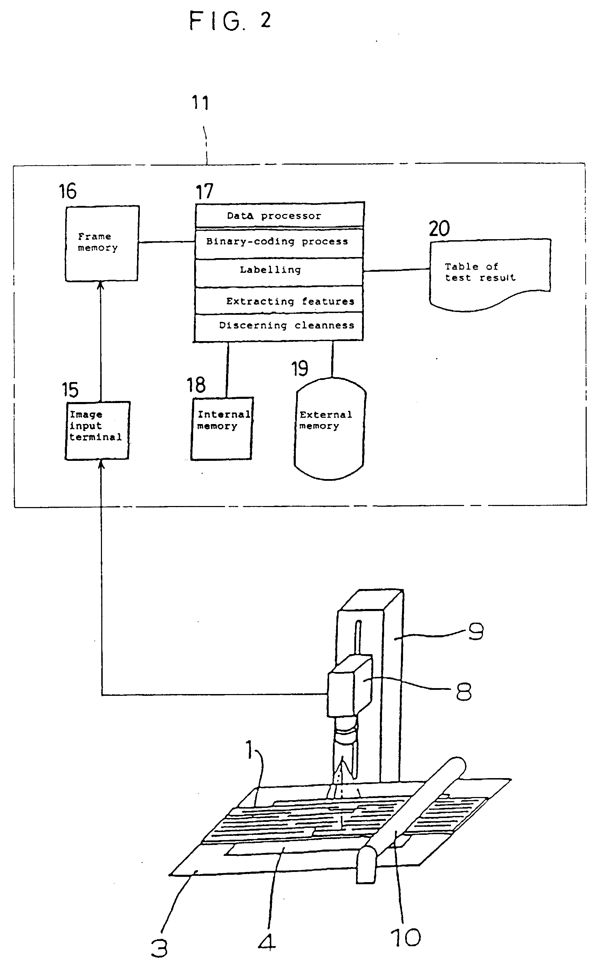

Fig. 2 presents further detail of the structure of the image processing unit 11. Structurally, the image processing unit 11 is composed of an image input terminal 15, a frame memory 16, a data processor 17, an internal memory 18, an external memory 19, and a system 20 available for printing the table of test results, respectively.

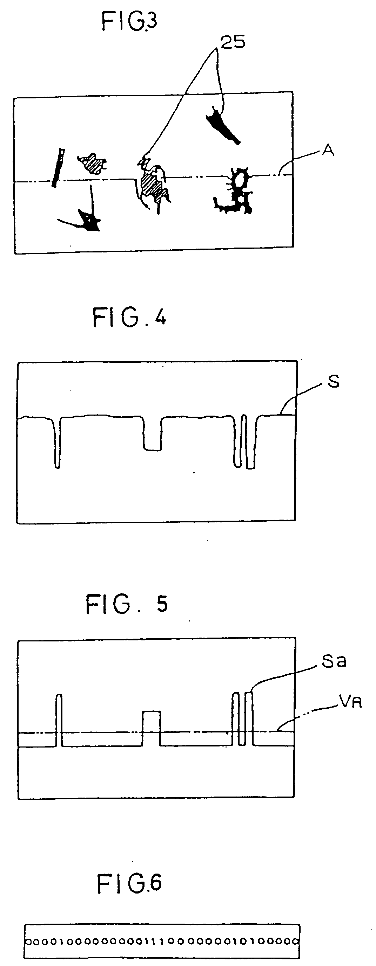

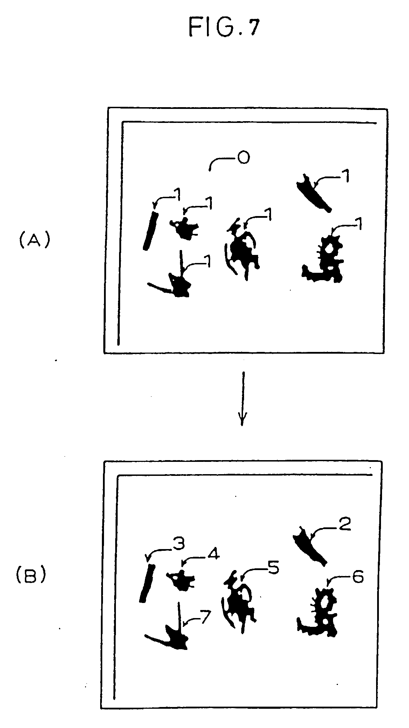

Fig. 3 illustrates a variety of images picked up by the image sensor 8. A variety of impurities and defects present in the sampled top sliber 1 are exposed to light beam permeated from the inspection window 4, and then, according to the kind of these impurities and defects, these are converted into black shadows containing depthwise gradation before eventually being caught by the image sensor 8. Next, the image containing depthwise gradation is converted into an image signal "S" (shown in Fig. 4) before eventually being delivered to the image processing unit 11. The image signal shown in Fig. 4 corresponds itself to the image containing depthwise gradation taken on line A. After being received by the image processing unit 11, the image signa; "S" is converted by an A/D converter into a multivalent image signal "Sa" (shown in Fig. 5) by providing each picture element with a certain depth. The frame memory 16 stores this multivalent image signal "SA". Next, the data processor 17 makes a comparison between the preset binary level and the multivalent image signal "Sa", and then generates a binary-coded image shown in Fig. 6. Next, the data processor 17 executes a pretreatment, in other words, a labelling operation, in order to secure features present in individual domains of those impurities and defects of the inspected top sliber 1 against the binary-coded image ("1" and "0" are respectively the values owned by figure) shown in Fig. 7A, and then, as shown in Fig. 7B, executes a numbering operation. Next, the data processor 17 computes the amount of features present in the numbered individual domains, and then stores the computed result in the internal memory 18. The apparatus embodied by the invention classifies cleanness of the inspected top sliber 1 based on the amount of features of defect.

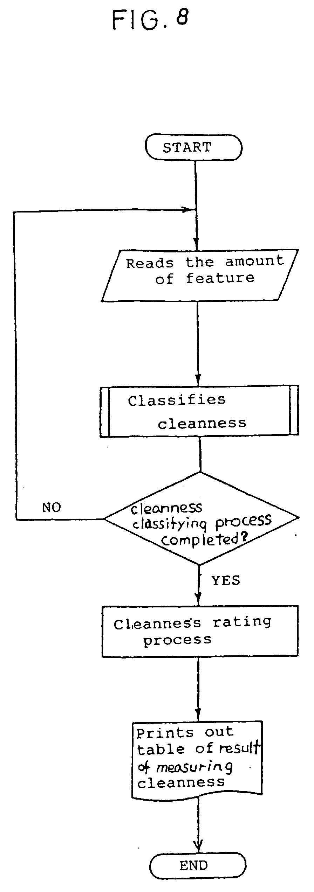

Fig. 8 presents an operating flowchart of those processes needed for discerning cleanness of the inspected top sliber 1. The data processor 17 reads the amount of feature from the internal memory 18, and then classifies cleanness. After fully completing the process for classifying cleanness, the data processor 17 rates the cleanness, and then prints the rated cleanness on a table designating the inspected results. If the process for classifying cleanness of the top sliber were not yet completed, then, the data processor 17 again reads the amount of feature before eventually classifying the cleanness of the inspected top sliber 1.

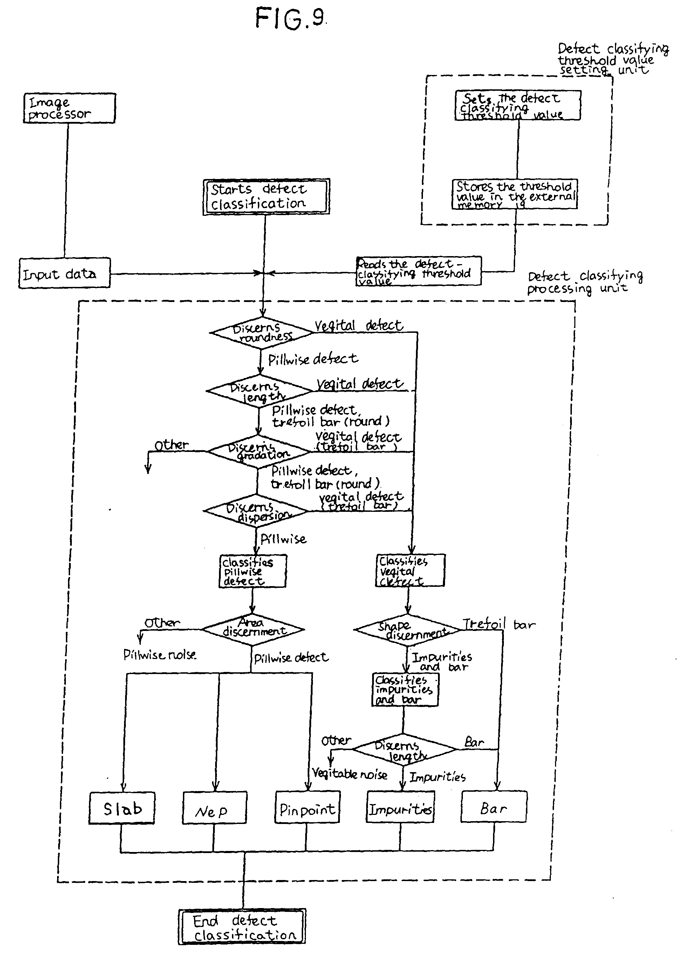

As is clear from the operating flowchart shown in Fig. 9, the data processor classifies defects of the inspected top sliber 1 according to respective check items based on the amount of feature stored in the internal memoery 18.

Fig. 9 presents the operating flowchart of the data processor 17 when classifying defects of the inspected wool top sliber. While the initial step of classification is underway, the data processor 17 discerns pillwise defect (including slab, nep, pinpoint, and round trefoil bar symptoms) from vegital defect (including impurities and bar symptoms). Roundness and slenderness are effectively made available for composing the amount of feature.

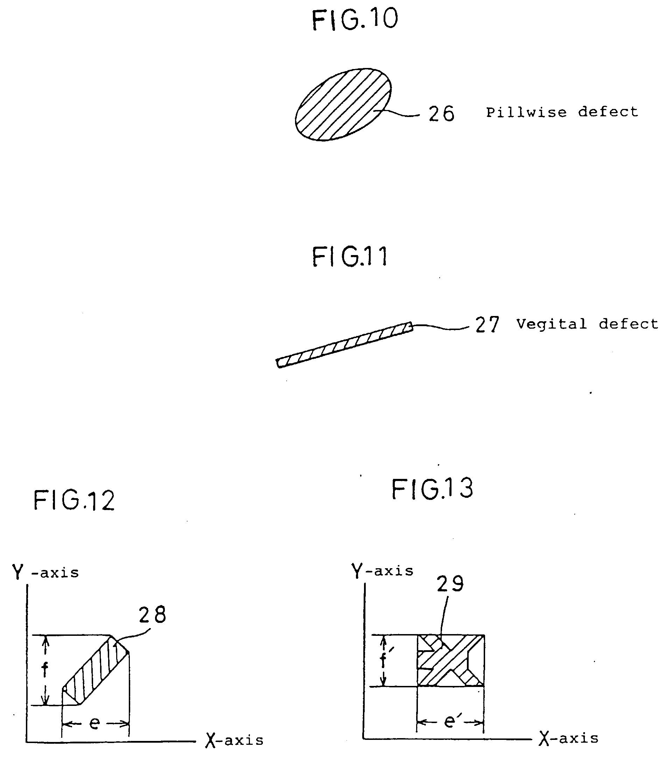

As shown in Figures 10 and 11, concretely, the data processor 17 discerns pillwise defect from vegital defect based on the rating of roundness and slenderness by effectively availing of those features including substantially round shape of the pillwise defect 26 and slender shape of the vegital defect 27. Roundness and slenderness are respectively rated by applying those expressions shown below. The data processor 17 executes classification of defects by comparing the computed values of roundness and slenderness to the predetermined reference value.

$$\text{Roundness} = 4\pi \text{ X (area per picture element) X (total number}$$

$$\text{of picture element)/(peripheral length)}^2$$

$$\text{Slenderness} = \text{(area per picture element) X (total number of}$$

$$\text{picture element)/(length of shadow projected}$$

$$\text{against axis X)}^2 + \text{(length of shadow projected}$$

$$\text{against axis Y)}^2$$

Availing of difference in the light-permeable amount between pills (including slab, nep, and pinpoint) and round trefoil bar, the data processor 17 discerns the pillwise defect from the round trefoil bar based on gadation discernment and dispersion discernment. Concretely, values of gradation and dispersion are computed by applying those expressions shown below. The data processor 17 classifies gradation and dispersion by comparing values of gradation and dispersion to the predetermined reference value.

$$\text{Gradation} = \text{(sum of depth levels)/(total number of picture element)}$$

$$\text{Dispersion} = (1/n) \Sigma Xi^2 - x^2$$

where

"n" designates the total number of picture elements;

$X_i$ designates depth levels of picture elements "i"; and

X designates gradation.

Pillwise defect is classified into slab, nep, and pinpoint according to the size. Therefore, pillwise defect is classified into three categories according to area, which is computed by applying the expression shown below.

$$\text{Area} = (\text{area per picture element}) \ X \ (\text{total number of picture element})$$

The data processor 17 classifies pillwise defect into slab, nep, and pinpoint in order of the size by making a comparison between the computed area value and the predetermined reference value. The data processor 17 further classifies the vegital defect into impurities, bar, and trefoil bar, by discerning these shapes. Concretely, shape is computed by applying the expression shown below.

$$\text{Shape} = (\text{peripheral length})/$$

$$\sqrt{(\text{lenth of shadow projected against axis X})^2 + (\text{length of shadow projected against axis Y})^2}$$

Fig. 12 graphically designates defect 28 caused by impurities. Assume that the defect 28 contains about 57 of peripheral length, 20 of the length "e" of shadow projected against axis X, and 20 of the length "f" of shadow projected against axis Y, then the shape of this defect 28 is substantially doubled as per the solution $57/\sqrt{20^2 + 20^2}$ of the above expression.

Fig. 13 graphically designates defect 29 caused by trefoil bar. Assume that this defect 29 contains about 97 of peripheral length, 20 of the length "e" of shadow projected against axis X, and 15 of the length "f" of shadow projected against axis Y, then, the shape of this defect 29 is quadrupled as per the solution $97/\sqrt{20^2 + 15^2}$ of the above expression. Concretely, the data processor 17 discernibly classifies defects into the one caused by the impurities and the other one caused by trefoil bar by making comparison between the above numerical values and the predetermined reference value. The data processor 17 discerns impurities from bar based on the measurement of length. Length is computed by applying the expression shown below.

$$\text{Length} = \sqrt{(\text{length of shadow projected against axis X})^2 + (\text{length of shadow projected against axis Y})^2}$$

Incidentally, the defect 20 caused by the impurities shown in Fig. 12 has about 28 of the length value as per the solution $\sqrt{20^2 + 20^2}$ of the above expression. The data processor 17 discerns this defect for classification by comparing it to the predetermined reference value. The trefoil bar cited above is eventually added to the category of bar.

In accordance with the operating flowchart shown in Fig. 8 and based on the result of classifying all defects, the data processor 17 executes the rating of cleanness of the inspected wool top sliber. The rated cleanness is effectively made available for composing quality standard of the top. The established quality standard is also effectively made available for composing basic data when spinning wool top sliber into yarns in the following processes.

Next, the method of classifying those defects by applying "fuzzy" reasoning is described below.

Initially, based on the definition of membership function, the data processor 17 establishes a specific range of those adjective expressions including "big", "intermediate", and "small" which are respectively applicable to the amount of features of defect including roundness, slenderness, area value, shape value, gradation value, length value, and dispersion value, and the other specific range of those adjective expressions including "high", "normal", and "low" for designating probability of being defect. The definition of membership function may be established in accordance with the judgement of those who are extremely skilled in the related art.

Next, using the range which is applicable to those adjective expressions like "big", "intermediate", and "small" cited above, the data processor 17 defines "fuzzy rule" for designating the range of the amount of features of defect in terms of the relationship between the combination and the probability of being specific defect, in other words, the data processor 17 defines the "fuzzy rule" which designates the relationship between the conditional terms like "if it were ---" and the conclusive terms like "probability of ---" for example.

Table 1 concretely designates the membership function and the "fuzzy rule" cited above. For example, if there were substantial roundness and slenderness, substantial area value, and in addition, more than intermediate rating of gradation, then, it is highly probable that the defect is caused by presence of slab, and therefore, there is less probability that the defect is caused by presence of pinpoint (p.p.) and noise. Neither high probability nor low probability of being nep, or trefoil bar, or bar, is present in the above defect, but the probability is rated to be normal. In this way, relative to the conditional section shown to the left of Table 1, the conclusion section is shown to the right.

Next, procedure for executing the "fuzzy reasoning" based on the above membership function and the "fuzzy rule" is described below.

Initially, input data is converted in correspondence with the amounts of respective features of a single defect. Next, adaptability to those adjective terms like "big", "intermediate", or "small", is computed from the membership function. Of those amounts of features of defect, if the objective shape were substantially circular, then the roundness is rated to be very close to 1. As mentioned earlier, the shape of pillwise defect is closer to circle than that of vegital defect, and thus, the difference between the pillwise defect and the vegital defect can be expressed by means of the rating of roundness. The rating of roundness is in a range from 0 to 1. This range is converted into 0 through 100(%). If the objective shaper were thinner, then the rating of slenderness approximates 0. Since the vegital defect has shape thinner than the pillwise defect, the difference between both defects can be designated. The rating of slenderness is in a range from 0 to 0.5. This range is converted into 0 through 100(%). Substantially, the gradation value represents the mean value of the depth of objects. Since more amount of light permeates the pillwise defect than the case of permeation through the vegital defect, the pillwise defect generates an image containing high-level density. The gradation value is in a range from 145 to 170. This range is also converted into 0 through 100(%). The dispersion value represents the unevenness of the depth of objects. Since the amount of light permeating plant and sliber differs, availing of substantial dispersion of depth level, precision in the classification of trefoil bar can be promoted even when sliber tangles trefoil bar. The dispersion value is in a range from 0 to 25(%). Like the above cases, this range is converted into 0 through 100(%). The shape value is incremental relative to the increase of shapes of objects by plural number. Since trefoil bar has shape being more complex than that of impurities and bars, availing of this complex shape, difference between trefoil bar and impurities/bar can effectively be designated. The shape value is in a range from 0 to 5. This range is also converted into 0 through 100(%). The area value designates areas of objects, in which pinpoint (p.p.), nep, and slab, are respectively classified according to own size, and thus, difference between these can be designated by means of area value. The area value is in a range from 0 to 100(%). This range is also converted into 0 through 100(%). The length value designates the length of objects. Availing of the length value, the data processor 17 discerns bar (having a minimum of 10mm of length) from impurities (having a minimum of 3mm and a maximum of 10mm of length). The length value is in a range from 0 to 10. This range is also converted into 0 through 100(%).

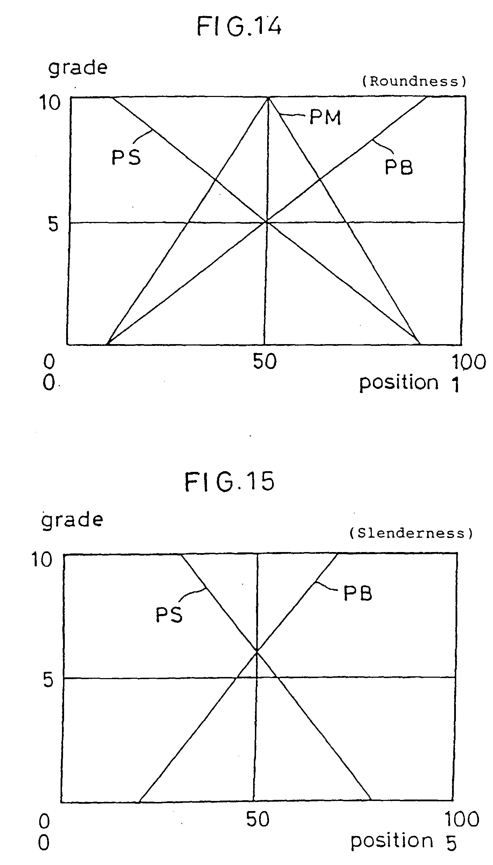

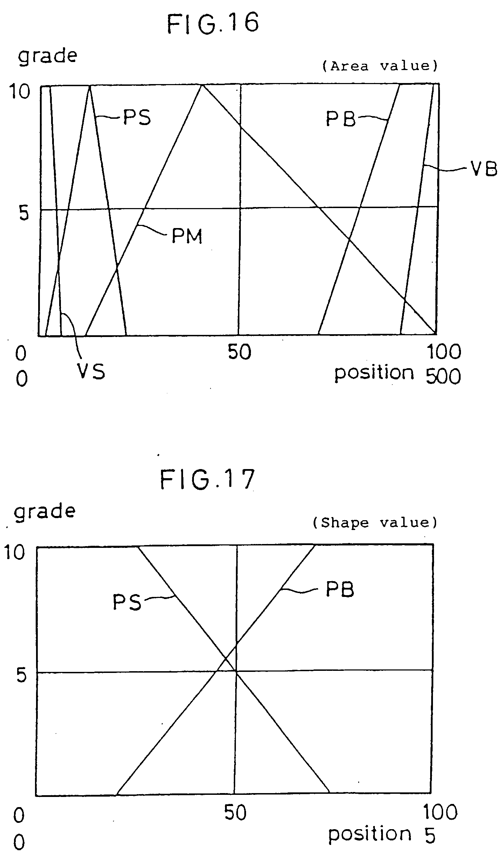

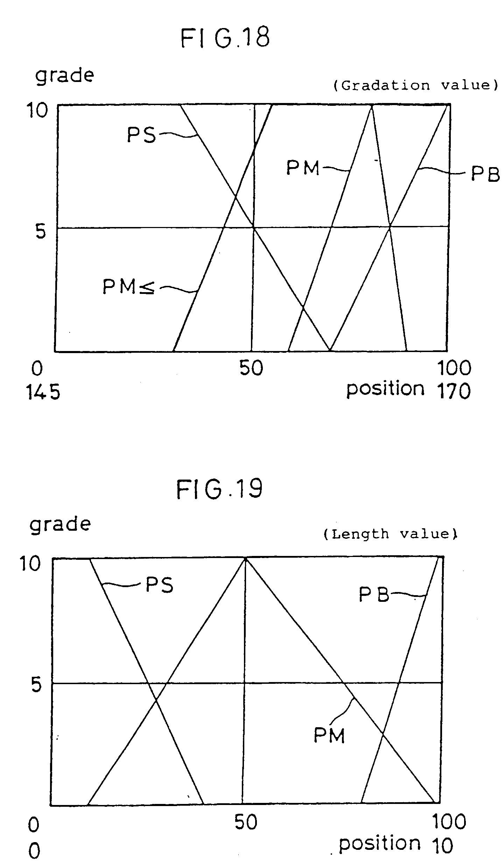

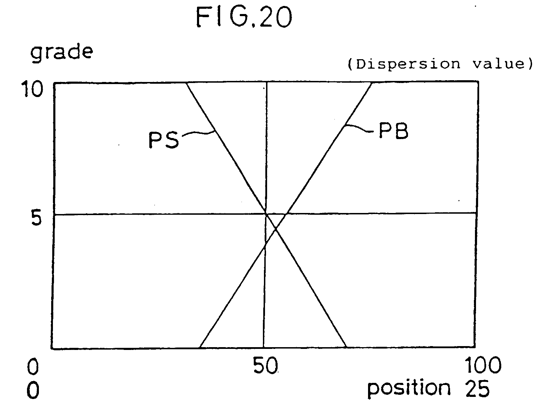

Fig. 14 through Fig. 20 graphically illustrate the membership function of roundness, slenderness, area value, shape value, gradation value, length value, and dispersion value. The label PS shown in these graphic charts designates "small". The label PM designates "intermediate". The label PB designates "big". The label VS designates "very small". The label VB designates "very big". The label PM< shown in the gradation value designates "being greater than intermediate".

TABLE 1   Contents of Fuzzy Rule

No.  Category of defect  Conditional section  Roundness
Slenderness  Area value   Shape value  Gradation value
Length value  Dispersion value

Pillwise defect  Big  Big  Big  Greater than intermediate
Big  Big  Intermediate  Big  Big  Big  Small  Big
Big  Big  Small  Big  Big  Big  Very small  Very big
Big  Big  Very small  Very big
Big  Big  Very small  Big  Big  Big  Very small  Big
BIg  Big  Small  Intermediate  Big  Big  Very small
Intermediate  Very big   Small

Vegital defect  Big  Big  Very small  Small  Small Small
Big  Small  Small  Small  Small  Small Big  Small  Small
Small Small  Small  Intermediate  Small  Big Very big
Intermediate  Big


Conclusion section  (probability of being defect of -- )
Slab  Nep  Pinpoint  Trefoil bar  Bar  Impurities  Noise
High  Normal  Low  Normal  Normal  Low  Normal  High  Normal
Low  Low  Low  Normal  High  Low  Normal  Low  Low High
Low  Normal  Normal  Low  Normal  Normal  Normal  High


TABLE 2

Amount of feature   Example of input data   Input value
Converted value (%)  Adaptability to words  ($0 \leqq$ Adaptability $\leqq 10$)
1. Roundness  2. Slenderness  3. Area value  4. Shape value
5. Gradation value  6. Length value   Very small  Small
Intermediate  Big  Very big  Very big

TABLE 3    Adaptability of Rule (Rule No. 11 for example)

Conditional section    Adaptability  Conclusion section

Adaptability   1. Roundness is small  Slab

2. Slenderness is small  Nep  3. Area value

4. Shape value is small  Pillwise noise

5. Gradation value is small  Probability of being trefoil bar
is low.

6. Length value is big  Probability of being bar is high.

Probability of being impurities
is normal.

Probability of being vegital noise
is low.

Adaptability of Rule No. 11  (Introduces minimum value of
adaptability in the Conditional
section)

TABLE 4  Results of defect classification

Defect detection rate    Defect discerning rate
(A/C X 100%)             (B/D X 100%)

Total number of data     Rate of achieving correct answer

Via fuzzy reasoning      Via conventional practice

A. Number of data which correctly discerned the defects
   from defect-free objects

B. Number of data which correctly discerned all defects
   from each other

C. Total number of data dealt by the data processor

D. Number of data which contained all the designated defects
   among those which are dealt by the data processor

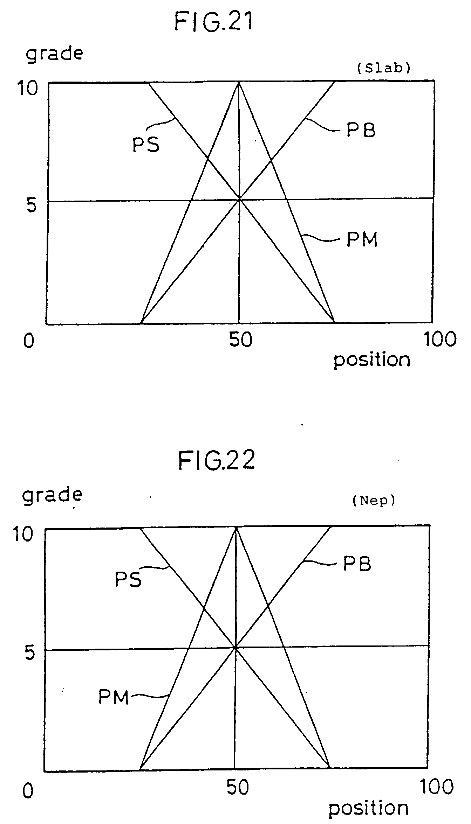

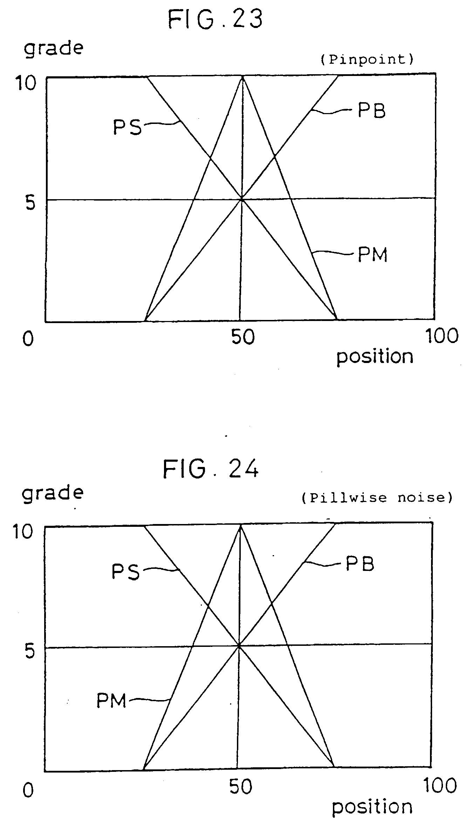

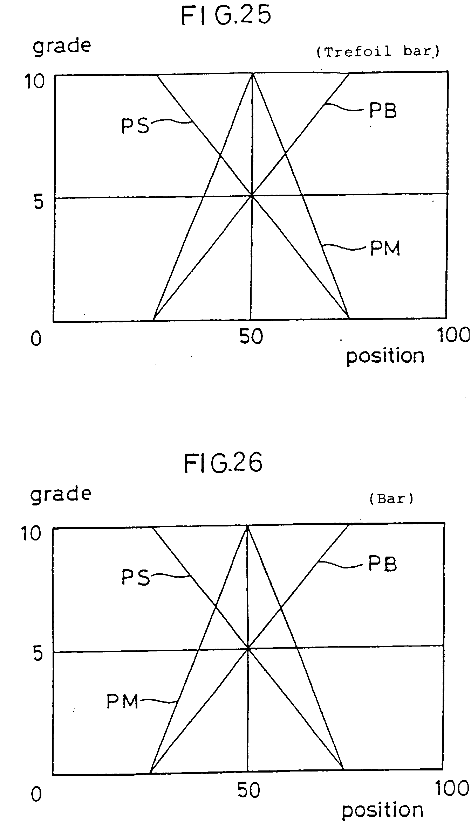

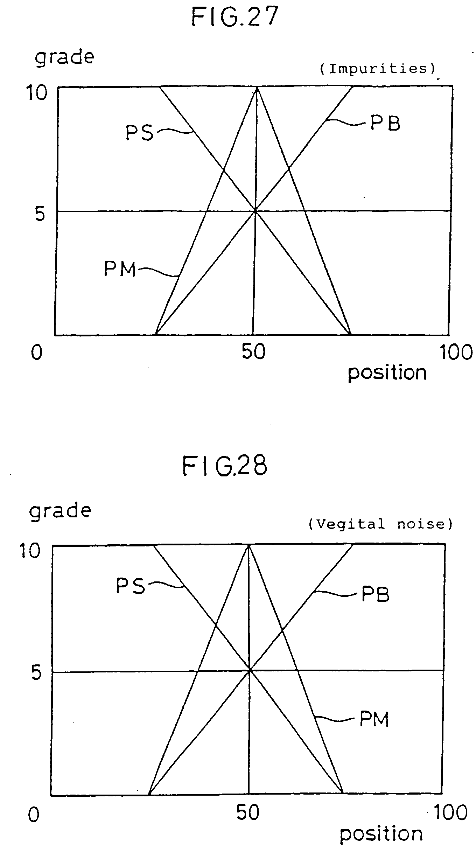

Fig. 21 through Fig. 28 graphically illustrate the membership function of slab, nep, pinpoint, pillwise noise, trefoil bar, bar, impurities, and vegital noise, which are respectively determined to be defect. The label PS designating probability indicates "low". The label PM indicates "normal". The other label PB indicates "high".

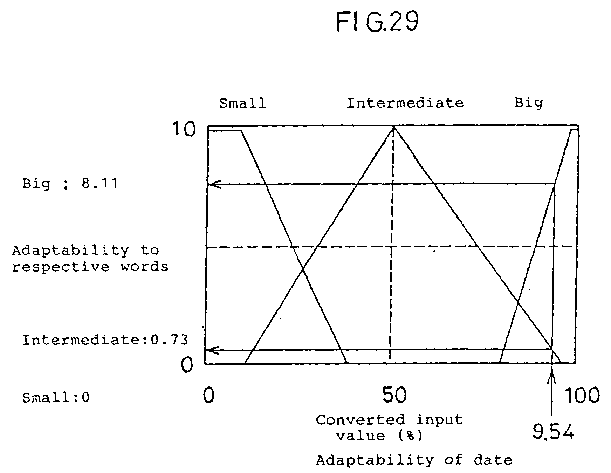

On receipt of those input data designating the roundness and other check items from the image processing signal, the data processor 17 computes adaptability of those adjective words "big", "intermediate", and "small" contained in the input values by applying those values converted from the input data. For example, if the input value of length value contained in an input data designating certain defect like bar were 9.54 and the converted value 95.4, then, as shown in Fig. 29 designating the membership function of the length value, the data processor 17 figures out the adaptability to the term "big" to be 8.11, adaptability to the term "intermediate to be 0.73, and the adaptability to the term "small" to be zero (the PS is free of intersecting point) from the vertical coordinate at the intersecting point of the vertical line of the input value 9.54 against inclined lines PB, PM, and PS. In the same way, the data processor 17 computes the adaptability to the amount of other features. Table 2 presents an example in which the data processor 17 computes adaptability to those adjective words in the course of dealing with a variety of defects containing 9.54 of the input length value, 0.24 of the input roundness value, 0.09 of the input slenderness value, 326.00 of the area value, 2.19 of the shape value, and 135.10 of the gradation value.

Next, the data processor 17 computes adaptability to respective rules by applying the adaptability to those adjective words designating the amount of features generated by applying those processes described above. For example, Table 3 designates the adaptability to the Rule No. 11 shown in Table 1. Concretely, as shown in Table 3, adaptability of the conclusion section (including the first through seventh conclusions) of the conclusion section of the Rule 11 is arranged to be the minimum value of adaptability in the conditional section shown to the left of Table 3. In this instance, the minimum value is 6.24.

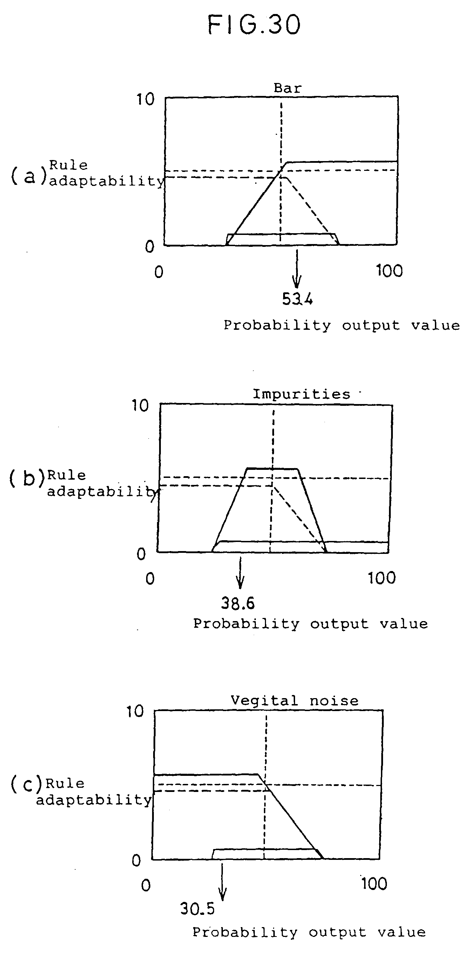

After determining the adaptability of the whole rules as described above, using the determined adaptability, the data processor 17 then executes an operation for computing minimum membership function of the corresponding conclusion section. Next, the data processor 17 executes an operation for computing maximum value to determine whether the probability of falling under respective defects is "high", or "normal", or "low". For example, by referring to the bar-defect determined by the fifth conclusion and based on the adaptability and the computed result, the data processor 17 computes minimum values of PB according to the adaptability value 6.24 of the Rule No. 11, PM according to the adaptability value 0.73 of the Rule No. 12, and PB according to the adaptability value 4.76 of the Rule No. 10. After synthesizing the computed values the data processor 17 establishes the relationship between the rule adaptability and the probability output value as shown in Fig. 30a. Likewise, Fig. 30a designates the synthesized result of the rule adaptability to defective impurities and Fig. 30b the synthesized result of the rule adaptability to vegital noise.

After completing those operations for computing maximum values, based on the yielded membership function, the data processor 17 computes confirmed values of probability of being defect. There are a variety of methods available for computing confirmed values of the probability including application of the center of gravity, center-computing method, height-computing method, and area-computing method. The data processor 17 computes confirmed values of the probability of being defect by applying more than one of those methods cited above. When computing the probability output values (confirmed values) of bar, impurities, and vegital noise based on the application of the center of gravity for example, the confirmed value of the bar defect is determined to be 53.4, the confirmed value of the impurities to be 38.6, and the confirmed value of the vegital noise to be 30.5. Based on these confirmed values, the data processor 17 concludes that the defect is substantially caused by presence of bar. In the event that the computation of confirmed value yields certain values being equal to each other in two kinds of defect then confirmable answer may be yielded by applying those different computing methods before eventually determining the confirmed value by accepting a majority decision.

Table 4 presents the results of the actually executed top inspection by applying the defect-classifying method via "fuzzy" reasoning. Table 4 also presents the result of inspecting defects based on a conventional method. As is clear from Table 4, although there is no substantial difference in the rate of detecting defect between those results yielded from the conventional method and the method embodied by the invention, the comparative result proves that the rate of yielding correct answer from the defect discernment based on the "fuzzy" reasoning introduced to the invented method has resulted in noticeable improvement.

As is clear from the above description, since the method embodied by the invention precisely classifies pillwise defect and vegital defect based on the roundness discernment and/or slenderness discernment, amount of data to be processed in the course of extracting features of defect sharply decreases. This in turn permits the method and apparatus embodied by the invention to significantly accelerate defect inspection with improved data processing efficiency.

Furthermore, as a result of the addition of a gradation discerning process and a dispersion discerning process to the roundness and slender discernments, the method and the apparatus embodied by the invention can precisely discern trefoil bar from slab, thus significantly promoting accuracy of the inspected results.

The method and the apparatus embodied by the invention precisely classify the pillwise defect further into those defects including slab, nep, and pinpoint, by applying area discernment, and yet, further classify the vegital defect into trefoil bar and those defects other than the trefoil bar like impurities and bar by applying shape discernment. The method and the apparatus embodied by the invention further classify impurities from bar by applying length discernment. This in turn permits the method and the apparatus embodied by the invention to finely classify defects per check item, thus offering practical advantage.

Furthermore, when classifying those defects based on the "fuzzy" reasoning, the method and the apparatus embodied by the invention do not respectively identify those defects based on an explicit threshold value, but the invented system solely reasons those defects by applying ambiguously defined domain, and therefore, the method and the apparatus embodied by the invention can achieve an overall result of judgement close to human judgement. This in turn permits the method and the apparatus embodied by the invention to very precisely discern those confusing plural defects like those which are actually present in the top sliber.

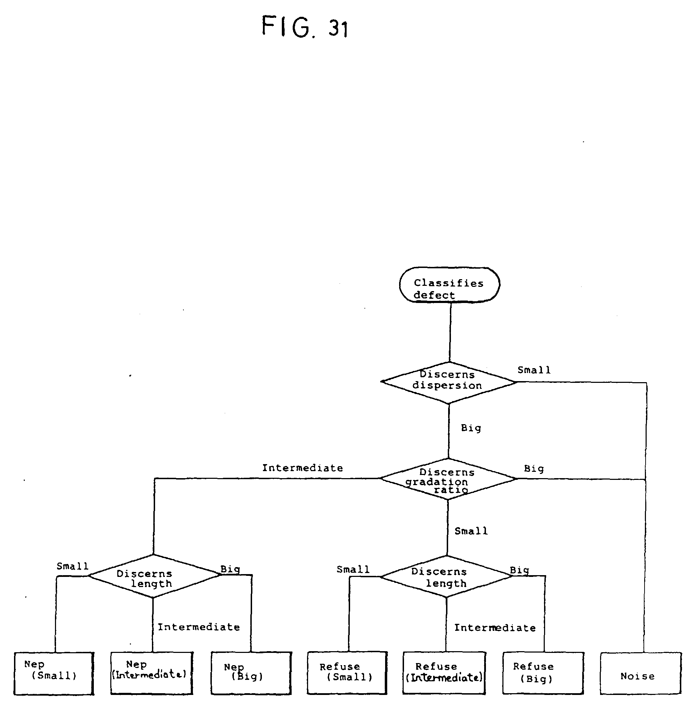

Fig. 31 presents an operating flowchart available for discerning defects of cotton sliber. The data processor 17 initially executes the dispersion discernment to disperse defects and noise, and then, discerns defects from each other based on the comparison of gradation. The data processor 17 discerns defects based on the comparison of gradation by checking the periphery of a specific area detected by means of binary code, and then, the periphery of the detected area and the gradation are compared to each other. The defect containing substantial gradation rate is identified to be noise. The defect containing negligible gradation rate is identified to be vegital mixture, whereas the defect containing modest gradation rate is identified to be pillwise defect. There are six kinds of defect inherent in cotton sliber including large, modest, and small vegital refuse, and large, modest, and small pillwise nep. However, there is little shapewise difference between vegital refuse and pillwise nep, and yet, unlike wool, there is no difference of size between these. Because of this, when executing a conventional method of classifying the cleanness of wool top, actually, refuse and nep are often incorrectly discerned from each other.

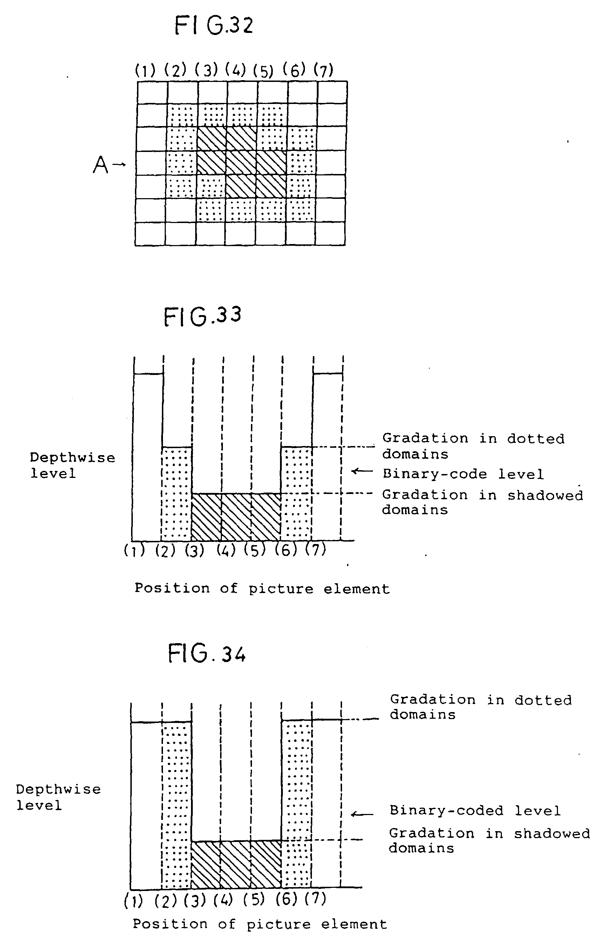

To solve this problem, it is quite effective for the defect inspecting system to introduce the method of discerning defects based on the comparison of gradation described above. Concretely, since pillwise defect is generated by tangled fibers, variation of depthwise level from the periphery of defect to the defective domain does not sharply occur unlike the case of vegital defect. For example, the image chart shown in Fig. 32 designates distribution of depthwise levels of picture elements on the line indicated by character A. Fig. 33 designates the distribution of depthwise levels of pillwise defect. Fig. 34 designates the distribution of depthwise levels of vegital defect. Based on these charts, the following expressions are established.

$$\text{Gradation} = (\text{sum of depthwise levels shown in shadowed}$$

$$\text{domain})/(\text{the number of picture elements shown}$$

$$\text{in the shadowed domain})$$

$$\text{Peripheral gradation ratio} = (\text{gradation})/[(\text{sum of depthwise}$$

$$\text{levels in the dotted domains})]/[(\text{the number}$$

$$\text{of picture elements in the dotted domains})]$$

where those values in brackets [ ] respectively designate gradation of domains in the periphery of defect.

As is clear from the above expressions, the less the difference of gradation between the defective domain and the peripheral domain, the closer the peripheral gradation ratio to value 1. Therefore, peripheral gradation ratio of pillwise defect exceeds that of vegital defect. The method and the apparatus embodied by the invention can precisely discern the pillwise defect from the vegital drect based on those processes described above. When computing the gradation ratio, the apparatus embodied by the invention initially establishes proper membership function, and then correctly discerns the pillwise defect from the vegital defect based on the "fuzzy" reasoning.

Industrial Applicability of the Invention

As is clear from the above description, the method and the apparatus for inspecting cleanness of top sliber embodied by the invention precisely and quickly analyzes cleanness of natural fibers like wool and/or cotton with perfect uniformity.

**Claims**

1. A method of inspecting cleanness of top sliver comprising the steps of:

    generating an image signal by picking up an image of said top sliver (1) with an image sensor (8); and discernibly classifying a defect into pillwise defect or vegetal defect mixed in said top sliver (1) based on said generated image signal;

said method being **characterized in that**

in the course of classifying said defects, a membership function (solely available for conditional section) for dealing with features of classified defects and the other membership function (solely available for conclusion section) for determining the probability of being defect against said classified defects are established, wherein said method also establishes a fuzzy rule by applying said conclusion section determining probability of being defect against those classified defects; and wherein said method further classifies defects based on fuzzy reasoning derived from said membership function (solely available for said conditional section), the other membership function (solely available for said conclusion section), and said fuzzy rule.

2. A method according to claim 1, wherein said step of discernibly classifying said defect into pillwise defect or vegetal defect is performed by applying roundness discernment and/or slenderness discernment.

3. A method according to claim 1, wherein said step of discernibly classifying said defect into pillwise defect or vegetal defect is performed by applying roundness discernment, slenderness discernment, gradation discernment, and dispersion discernment.

4. A method according to claim 3, further comprising the steps of

discernibly classifying said pillwise defect further into defects including slab, nep, and pinpoint defects, by applying area discernment, and
discernibly classifying said vegetal defect further into trefoil bar defects and defects other than said trefoil bar defects like impurities and bar, by applying shape discernment, and yet, further classifying said impurities and bar by applying length discernment.

5. A method according to claim 1, wherein said step of discernibly classifying said defect into pillwise defect or vegetal defect is performed by applying gradation discernment and/or gradation ratio discernment;

said method further comprising a step of further classifying said pillwise defect and said vegetal defect into respective defects by applying length discernment.

6. An apparatus for inspecting cleanness of top sliver comprising:

a gill box (2) which uniformly spreads a top sliver (1) and then externally delivers it;
a plurality of guide rollers (13, 13a) which respectively guide said spread top sliver (1);
an illuminating unit (6) which irradiates said spread top sliver (1) with a light beam;
an image sensor (8) which picks up an image of said spread top sliver (1) and generates an image signal;
a contact-free electrostatic preventing unit (30) which is installed in the vicinity of said plurality of guide rollers (13, 13a) and eliminates static charge from said spread top sliver (1); and
an image processing unit (11) which processes said image signal generated by said image sensor (8); said apparatus being **characterized in that**
in the course of classifying said defects, said image processing unit (11) establishes a membership function (solely available for conditional section) for dealing with features of classified defects and the other membership function (solely available for conclusion section) for determining the probability of being defect against said classified defects, wherein said image processing unit (11) also establishes a fuzzy rule by applying said conclusion section determining probability of being defect against those classified defects; and wherein said image processing unit (11) further classifies defects based on fuzzy reasoning derived from said membership function (solely available for said conditional section), the other membership function (solely available for said conclusion section), and said fuzzy rule.

**Patentansprüche**

1. Verfahren zum Prüfen der Sauberkeit von Kammzugband mit den Schritten:

- Erzeugen eines Bildsignals durch Aufnehmen eines Bilds eines Kammzugbands **(1)** mit einem Bildsensor **(8)**, und
- unterscheidbares Klassifizieren eines Fehlers auf der Grundlage des erzeugten Bildsignals in einen Pillingfehler oder einen Pflanzenrestfehler, welche in dem Kammzugband **(1)** vermischt sind, wobei das Verfahren

**dadurch gekennzeichnet** ist, daß im Verlauf des Klassifizierens der Fehler eine Zugehörigkeitsfunktion (nur verfügbar für Bedingungsteil) für die Behandlung der Merkmale der klassifizierten Fehler und die andere Zugehörigkeitsfunktion (nur verfügbar für den Folgerungsteil) zum Bestimmen der Wahrscheinlichkeit des Vorliegens des Fehlers gegenüber den klassifizierten Fehlern begründet werden, wobei das Verfahren auch eine Fuzzy-Regel durch Anwenden des Folgerungsteils zum Bestimmen der Wahrscheinlichkeit des Vorliegens des Fehlers gegenüber jenen klassifizierten Fehlern begründet, und wobei das Verfahren ferner die Fehler auf der Grundlage der Fuzzy-Argumentation klassifiziert, welche von der Zugehörigkeitsfunktion abgeleitet ist (nur verfügbar für den Bedingungsteil), der anderen Zugehörigkeitsfunktion (nur verfügbar für den Folgerungsteil) und der Fuzzy-Regel.

2. Verfahren gemäß Anspruch 1, wobei der Schritt des unterscheidbaren Klassifizierens des Fehlers in den Pillingfehler oder den Pflanzenrestfehler durch Anwenden der Unterscheidung des Rundheitsgrads und/oder der Unterscheidung des Schlankheitsgrads ausgeführt wird.

3. Verfahren gemäß Anspruch 1, wobei der Schritt des unterscheidbaren Klassifizierens des Fehlers in den Pillingfehler oder den Pflanzenrestfehler durch Anwenden der Unterscheidung des Rundheitsgrads, der Unterscheidung des Schlankheitsgrads, der Unterscheidung der Abstufung und der Unterscheidung der Streuung ausgeführt wird.

4. Verfahren gemäß Anspruch 3, welches ferner die Schritte aufweist:

   - unterscheidbares Klassifizieren des Pillingfehlers ferner in Fehler, welche Flachkörper-, Noppen- und Pinpoint-Fehler einschließen, durch Anwenden der Unterscheidung der Fläche, und
   - unterscheidbares Klassifizieren des Pflanzenrestfehlers ferner in Kleeblatt-Langkörper-Fehler und andere Fehler als Kleeblatt-Langkörper-Fehler, wie Verunreinigungen und Langkörper, durch Anwenden der Unterscheidung der Form, und noch weiteres Klassifizieren der Verunreinigungen und des Langkörpers durch Anwenden der Unterscheidung der Länge.

5. Verfahren gemäß Anspruch 1, wobei der Schritt des unterscheidbaren Klassifizierens des Fehlers in den Pillingfehler oder den Pflanzenrestfehler durch Anwenden der Unterscheidung der Abstufung und/oder der Unterscheidung des Abstufungsverhältnisses ausgeführt wird, und das Verfahren ferner einen Schritt des weiteren Klassifizierens des Pillingfehlers und des Pflanzenrestfehlers in die jeweiligen Fehler durch Anwenden der Unterscheidung der Länge aufweist.

6. Vorrichtung zum Prüfen der Sauberkeit des Kammzugbands, welche aufweist:

   - eine Nadelstabstrecke (2), welche ein Kammzugband (1) gleichmäßig ausbreitet und es dann nach außen austrägt,
   - eine Vielzahl von Führungswalzen (13, 13a), welche jeweils das ausgebreitete Kammzugband (1) führen,
   - eine Beleuchtungseinheit (6), welche das ausgebreitete Kammzugband (1) mit einem Lichtstrahl beleuchtet,
   - einen Bildsensor (8), welcher ein Bild des ausgebreiteten Kammzugbands (1) aufnimmt und ein Bildsignal erzeugt,
   - eine kontaktlose elektrostatische Verhinderungseinheit (30), welche in der Nähe der Vielzahl von Führungswalzen (13, 13a) angeordnet ist und die elektrostatische Ladung des ausgebreiteten Kammzugbands (1) neutralisiert, und
   - eine Bildverarbeitungseinheit (11), welche das durch den Bildsensor (8) erzeugte Bildsignal verarbeitet, wobei die Vorrichtung

   **dadurch gekennzeichnet** ist, daß im Verlauf des Klassifizierens der Fehler die Bildverarbeitungseinheit (11) eine Zugehörigkeitsfunktion begründet (nur verfügbar für den Bedingungsteil), zum Behandeln der Merkmale der klassifizierten Fehler, und die andere Zugehörigkeitsfunktion (nur verfügbar für den Folgerungsteil) zum Bestimmen der Wahrscheinlichkeit des Vorliegens des Fehlers gegenüber den klassifizierten Fehlern, wobei die Bildverarbeitungseinheit (11) auch eine Fuzzy-Regel durch Anwenden des Folgerungsteils begründet, zum Bestimmen der Wahrscheinlichkeit des Vorliegens des Fehlers gegenüber jenen klassifizierten Fehlern, und wobei die Bildverarbeitungseinheit (11) ferner die Fehler auf der Grundlage der Fuzzy-Argumentation, abgeleitet aus der Zugehörigkeitsfunktion (nur verfügbar für den Bedingungsteil), der anderen Zugehörigkeitsfunktion (nur verfügbar für den Folgerungsteil) und der Fuzzy-Regel klassifiziert.

**Revendications**

1.  Procédé d'inspection de la netteté d'un ruban, comprenant les étapes consistant à :

    -   produire un signal d'image par saisie d'une image dudit ruban (1) avec un capteur d'image (8), et
    -   classer de manière reconnaissable un défaut en défauts de type globules et en défauts végétaux mélangés dans ledit ruban (1), en se basant sur ledit signal d'image produit,

    ledit procédé étant caractérisé en ce que, lors de ladite classification des défauts, il est établi une première fonction d'appartenance (disponible uniquement pour la partie conditionnelle) qui s'intéresse aux particularités des défauts classés et une autre fonction d'appartenance (disponible uniquement pour la partie conclusion) qui détermine la probabilité de défectuosité par rapport auxdits défauts classés, ledit procédé établissant aussi une règle floue en appliquant à ladite partie conclusion une détermination de la probabilité de défectuosité par rapport auxdits défauts classés, ledit procédé classant en outre les défauts en se basant sur un raisonnement flou dérivé de ladite première fonction d'appartenance (disponible uniquement pour la partie conditionnelle), de ladite autre fonction d'appartenance (disponible uniquement pour la partie conclusion) et de ladite règle floue.

2.  Procédé selon la revendication 1, dans lequel ladite étape de classification reconnaissable des défauts en défauts de type globules et en défauts végétaux est réalisée par application d'une reconnaissance de rotondité et/ou de minceur.

3.  Procédé selon la revendication 1, dans lequel ladite étape de classification reconnaissable des défauts en défauts de type globules et en défauts végétaux est réalisée par application d'une reconnaissance de rotondité, d'une reconnaissance de minceur, d'une reconnaissance de gradation et d'une reconnaissance de dispersion.

4.  Procédé selon la revendication 3, comprenant en outre les étapes consistant à :

    -   classer en outre de manière reconnaissable lesdits défauts de type globules en défauts comprenant des défauts de type plaque, noeud et trous d'épingle par application d'une reconnaissance de superficie, et
    -   classer en outre de manière reconnaissable lesdits défauts végétaux en défauts de type brins de trèfle et en défauts autres que lesdits brins de trèfle comme des impuretés et des brins par application d'une reconnaissance de forme, et classer en outre lesdites impuretés et lesdits brins par application d'une reconnaissance de longueur.

5.  Procédé selon la revendication 1, dans lequel ladite étape de classification reconnaissable des défauts en défauts de type globules et en défauts végétaux est réalisée par application d'une reconnaissance de gradation et/ou d'une reconnaissance de taux de gradation, ledit procédé comprenant en outre une étape consistant à classer en outre lesdits défauts de type globules et lesdits défauts végétaux en défauts respectifs par application d'une reconnaissance de longueur.

6.  Appareil d'inspection de la netteté d'un ruban, comprenant:

    -   une gill-box (2) qui étale uniformément un ruban (1) puis le délivre à l'extérieur,
    -   une pluralité de rouleaux de guidage (13, 13a) qui guident respectivement ledit ruban étalé (1),
    -   une unité d'éclairage (6) qui envoie sur ledit ruban étalé (1) un faisceau lumineux,
    -   un capteur d'image (8) qui saisit une image dudit ruban étalé (1) et produit un signal d'image,
    -   une unité anti-électricité statique (30) sans contact, qui est installée au voisinage de ladite pluralité de rouleaux de guidage (13, 13a) et qui élimine les charges électrostatiques dudit ruban étalé (1), et
    -   une unité (11) de traitement d'image qui traite ledit signal d'image produit par ledit capteur d'image (8),

    ledit appareil étant caractérisé en ce que, lors de ladite classification des défauts, ladite unité (11) de traitement d'image établit une première fonction d'appartenance (disponible uniquement pour la partie conditionnelle) qui s'intéresse aux particularités des défauts classés et une autre fonction d'appartenance (disponible uniquement pour la partie conclusion) qui détermine la probabilité de défectuosité par rapport auxdits défauts classés, ladite unité (11) de traitement d'image établissant aussi une règle floue en appliquant à ladite partie conclusion une détermination de la probabilité de défectuosité par rapport auxdits défauts classés, ladite unité (11) de traitement classant en outre les défauts en se basant sur un raisonnement flou dérivé de ladite première fonction d'appartenance (disponible uniquement pour la partie conditionnelle), de ladite autre fonction d'appartenance (disponible uniquement pour la partie conclusion) et de ladite règle floue.

# FIG. 1

Image processing unit

11

14

8 Image sensor

9

3 Inspection table

30

31a

2

12

12a

1

4

6 10 13 31b

13a

# FIG. 2

11

16

| 17 |
| --- |
| Data processor |
| Binary-coding process |
| Labelling |
| Extracting features |
| Discerning cleanness |

| 16 |
| --- |
| Frame memory |

20

| Table of test result |

| 15 |
| --- |
| Image input terminal |

| 18 |
| --- |
| Internal memory |

19

| External memory |

9

8

1

3    4    10

## FIG.3

## FIG.4

## FIG. 5

## FIG.6

## FIG. 7

# FIG. 8

```
        ┌─────────────┐
        │    START    │
        └──────┬──────┘
               │
   ┌───────────┼
   │           ▼
   │    ╱─────────────────╲
   │   ╱  Reads the amount ╲
   │   ╲    of feature     ╱
   │    ╲─────────────────╱
   │           │
   │           ▼
   │   ║┌─────────────────┐║
   │   ║│   Classifies    │║
   │   ║│    cleanness    │║
   │   ║└─────────────────┘║
   │           │
   │           ▼
   │         ╱───╲
   │   NO  ╱  cleanness ╲
   └──────   classifying process
           ╲  completed?  ╱
             ╲──────╱
               │ YES
               ▼
        ┌─────────────────┐
        │ Cleanness rating│
        │     process     │
        └─────────┬───────┘
                  │
                  ▼
        ┌─────────────────┐
        │ Prints out      │
        │ table of result │
        │ of measuring    │
        │ cleanness       │
        └─────────┬───────┘
                  │
                  ▼
        ┌─────────────┐
        │     END     │
        └─────────────┘
```

19

# FIG.9

## FIG.10

26    Pillwise defect

## FIG.11

27   Vegital defect

## FIG.12

Y-axis

28

f

e

X-axis

## FIG.13

Y-axis   29

f'

e'

X-axis

21

## FIG.14

## FIG.15

# FIG.16

grade

(Area value)

# FIG.17

grade

(Shape value)

# FIG.18

# FIG.19

# FIG.20

grade

(Dispersion value)

PS    PB

position 25

## FIG.21

grade                                    (Slab)

position

## FIG.22

grade                                    (Nep)

position

# FIG.23

grade

(Pinpoint)

PS, PB, PM curves; x-axis position 0–100, y-axis grade 0–10

# FIG.24

grade

(Pillwise noise)

PS, PB, PM curves; x-axis position 0–100, y-axis grade 0–10

## FIG.25

## FIG.26

## FIG.27

## FIG.28

FIG.29

Small       Intermediate       Big

Big : 8.11

Adaptability to
respective words

Intermediate:0.73

Small:0

Converted input
value (%)

9.54

Adaptability of date

# FIG.30

(a) Rule adaptability

Bar

10

0

0        53.4        100

Probability output value

(b) Rule adaptability

Impurities

10

0

0        38.6        100

Probability output value

(c) Rule adaptability

Vegital noise

10

0

0        30.5        100

Probability output value

# FIG. 31

## FI G.32

## FI G.33

Position of picture element

## FIG.34

Position of picture element